# EUROPÄISCHE PATENTSCHRIFT

(11) **EP 0 388 580 B1**
(45) Veröffentlichungstag und Bekanntmachung des Hinweises auf die Patenterteilung: **14.09.1994**
(21) Anmeldenummer: 90100498.6
(22) Anmeldetag: 11.01.1990
(51) Int. Cl.: A61B 1/04

(54) **Bildgebendes Aufnahme- und/oder Übertragungsmittel**
Recording and transmission means providing an image display
Appareil d'enregistrement et de transmission pourvu d'un dispositif de reproduction d'images

(30) Priorität: 20.03.1989 DE 3909090
(43) Veröffentlichungstag der Anmeldung: 26.09.1990
(73) Patentinhaber: Richard Wolf GmbH, 75438 Knittlingen (DE)
(72) Erfinder: Ams, Felix, D-7539 Kämpfelbach (DE)
(74) Vertreter: Wilcken, Thomas, Dipl.-Ing.

(56) Entgegenhaltungen:
- GB-A- 2 105 196
- US-A- 4 722 000
- PATENT ABSTRACTS OF JAPAN vol. 10, no. 157 (P-464)(2213) 6 Juni 1986,& JP-A-61 011648 (MATSUSHITA DENKI SANGYO K.K.) 20 Januar 1986,
- FUNKSCHAU. vol. 53, no. 14, Juli 1981, MUNCHEN DE Seiten 62 - 64;"Gebrauchstest NV-7000E; übersichtliche Bedienung"

## Beschreibung

Die Erfindung geht aus von einem bildgebenden Aufnahme- und/oder Übertragungsmittel, wie Endoskope, Videoskope, Techno-Endoskope, Endo-Kameras oder dergleichen, dessen bzw. deren abgedichteter Hohlraum an eine Einrichtung zum Erzeugen eines Überdrucks darin anschließbar ist. Ein solches bildgebendes Mittel ist in der DE-A-33 46 850 beschrieben.

Bildgebende Aufnahme- und/oder Übertragungsmittel, wie Endoskope, Videoskope, Techno-Endoskope, Endo-Kameras oder dergleichen, besitzen abgedichtete Hohlräume, in die durch unsachgemäße Anwendung oder Beschädigungen Luftfeuchtigkeit oder Flüssigkeit eindringt, wodurch es unter Umständen zu erheblichen Beeinträchtigungen der Funktion und/oder auch zu einem vollständigen Funktionsausfall kommen kann, so daß teure Reparaturen und erhebliche Arbeitsaufwände erforderlich werden.

Es ist aus der GB-A-2 105 196 bekannt, bei endoskopischen Eingriffen, wenn explosive Gase oder Gasgemische vorhanden sein können, z. B. im Dickdarm, eine gezielte Überwachung des Gasgemisches bzw. seiner möglichen Explosivität durchzuführen. Hierzu werden von einem Gassensor ermittelte Meßwerte so verarbeitet, daß das verwendete elektrochirurgische Instrument abgeschaltet und das Gasgemisch mit Luft verdünnt wird, sobald ein kritischer Wert des Gasgemisches erreicht worden ist. Dabei wird durch eine Einrichtung die Temperatur und Luftfeuchtigkeit des Gasgemisches berücksichtigt, um Meßfehler des Sensors zu vermeiden.

Aus FUNKSCHAU, Band 53, Nr. 14, Juli 1981, München S. 62-64 "Gebrauchstest NV-7000 E; übersichtliche Bedienung" ist bekannt, die Inbetriebnahme eines Videorecorders zu verhindern, Wenn ein Taudetektor erhöhte Luftfeuchtigkeit detektiert.

Um etwaige Schäden an bildgebenden Instrumenten durch Eindringen von Luftfeuchtigkeit in einen Hohlraum der Instrumente festzustellen, geht man nach der DE-OS 33 46 850 so vorgegangen, daß das jeweilige Instrument, nachdem sein Hohlraum nach Anschließen des Instruments an eine Druckgaszuführeinrichtung mit einem Überdruck versehen worden ist, in eine Flüssigkeit eingetaucht wird, so daß aus dem beschädigten Hohlraum Luft in Form von Luftblasen austritt, womit die Schadstelle genau festgestellt und der Schaden behoben werden kann.

Eine derartige Feststellung von Schäden hängt mehr oder weniger stark von Zufällen ab, weil der Benutzer solche Prüfungen nicht gezielt in regelmäßigen Abständen durchführt.

Die Aufgabe der Erfindung besteht darin, ein bildgebendes Aufnahme- und/oder Übertragungsmittel so auszubilden, daß bereits das Eindringen von sehr geringen Mengen an Luftfeuchtigkeit von dem Benutzer erkannt und die auftretende Beschädigung anschließend behoben werden kann.

Diese Aufgabe wird durch die Merkmale des Anspruches 1 gelöst.

Durch diese Lösung gelangt von dem Sensor oder den Sensoren beim Eindringen der Luftfeuchtigkeit in den abgedichteten Hohlraum des bildgebenden Aufnahme- und/oder Übertragungsmittels ein Signal an die nachgeschaltete Elektronik, die dann nach Erreichen eines frei wählbaren Grenzwertbereiches in Abhängigkeit des vorwählbaren Feuchtigkeitsgrenzwertes den Istwert oder die Differenz zum maximal zulässigen Grenzwert anzeigt und bei Überschreiten des Grenzwertes ein Signal abgibt, worauf die Überdruckpumpe zur Vermeidung von Beschädigungen den Innenraum des bildgebenden Aufnahme- und/oder Übertragungsmittels mit Überdruck beaufschlagt, so daß keine weitere Feuchtigkeit oder Flüssigkeit in den Innenraum eindringen kann.

Die Erfindung ist nachstehend anhand der Zeichnung erläutert. Es zeigen:

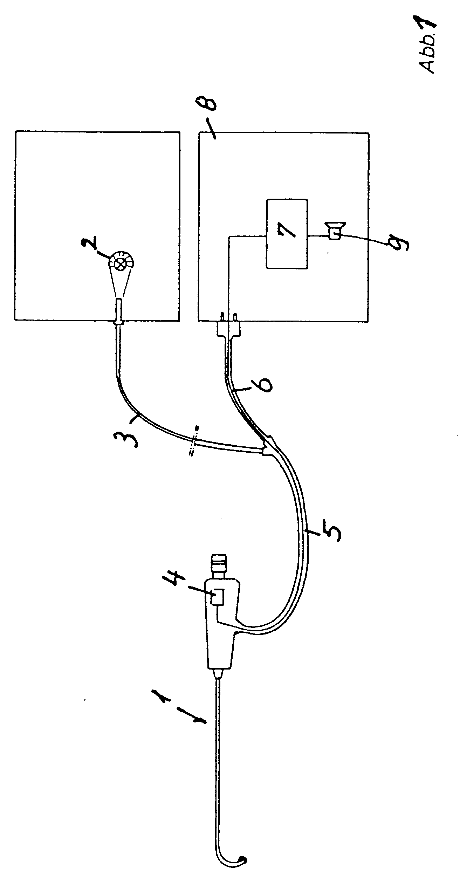

- Figur 1: schematisch ein flexibles Endoskop mit einer Lichtquelle und einer elektronischen Einrichtung zur Überwachung der Luftfeuchtigkeit in einem Hohlraum des Endoskops,
- Figur 2: das gleiche Schema, jedoch mit einer an das Endoskop angeschlossenen Endo-Videokamera,

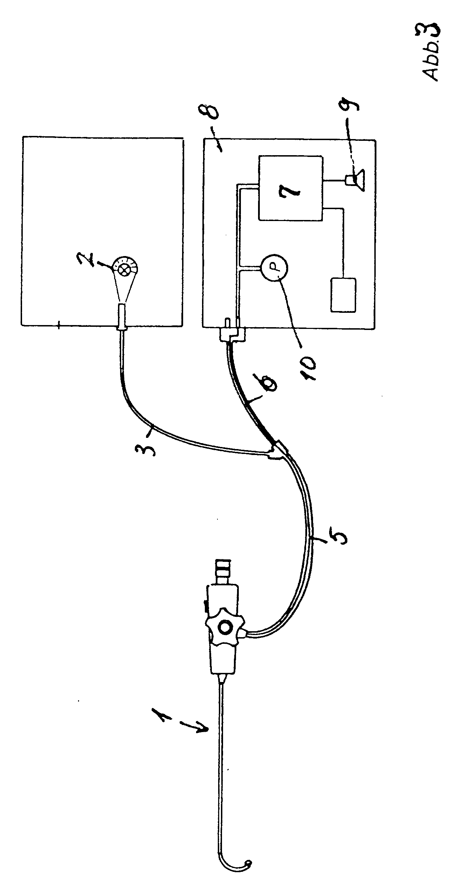

- Figur 3: ein der Fig. 1 entsprechendes Aufbauschema mit einer in der elektronischen Überwachungseinrichtung integrierten Pumpe zur Erzeugung eines Luftüberdruckes.

An das bildgebende Instrument in Form eines Endoskops 1 ist eine Lichtquelle 2 angeschlossen, deren Licht über einen Lichtleiter 3 durch das Endoskop hindurch zur Beleuchtung eines Gegenstandes dient.

In einem abgedichteten Hohlraum des Endoskops 1 ist ein schon auf geringe Feuchtigkeitsmengen ansprechender, bekannter Sensor 4 angeordnet, der über Anschlußleitungen 5 und 6 mit einer Auswerteelektronik 7 einerelektronischen Überwachungseinrichtung 8 verbunden ist. Durch die Auswerteelektronik 7 wird der durch den Sensor 4 bei erhöhter Luftfeuchtigkeit in dem Endoskophohlraum gemessene Istwert mit einem maximal zulässigen Grenzwert verglichen und, sofern der Istwert einen vorwählbaren Grenzbereich erreicht, der Istwert oder die Differenz zum maximal zulässigen Grenzwert angezeigt und bei Überschreiten des Grenzwertes von einer Signaleinrichtung 9 ein akustisches Signal abgegeben sowie zur Vermeidung des weiteren Eindringens von Feuchtigkeit in den Endoskopinnenraum eine mit diesem Innen raum in Verbindung stehende Überdruckpumpe 10 in Betrieb genommen.

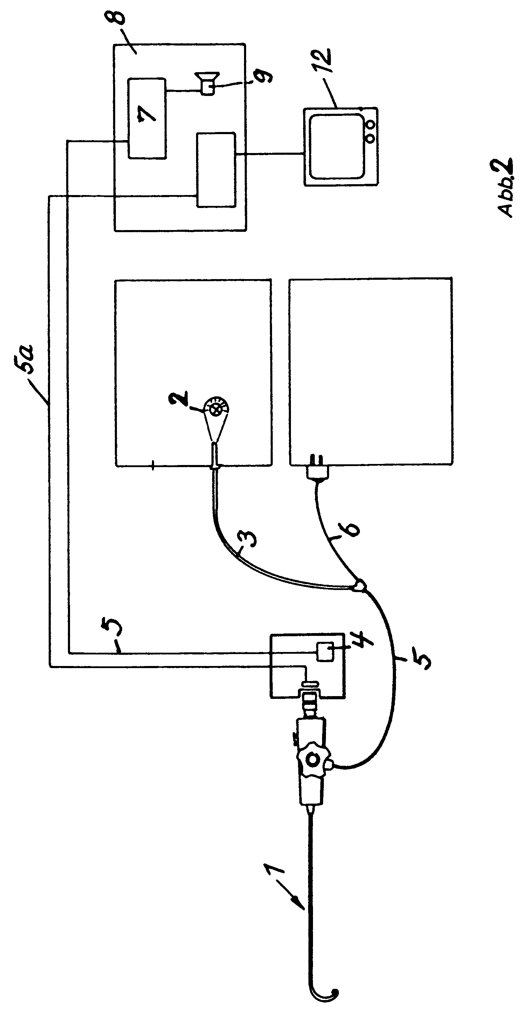

Figur 2 zeigt anstelle eines Endoskops eine Endo-Kamera mit einem in ihrem Innenraum angeordneten Sensor 4, der dann wieder mit der Auswerteelektronik der elektronischen Überwachungseinrichtung 8 zusammenarbeitet, wobei die Kamera über eine Leitung 5a an einen Monitor 12 oder Bildschirm abgeschlossen ist.

## Patentansprüche

1. Bildgebendes Aufnahme- und/oder Übertragungsmittel (1), wie ein Endoskop, ein Techno-Endoskop, ein Videoskop oder eine Endo-Kamera, dessen bzw. deren abgedichteter Hohlraum an eine Einrichtung zum Erzeugen eines Überdruckes darin anschließbar ist, dadurch gekennzeichnet, daß in dem abgedichteten Hohlraum des Aufnahme- und/oder Übertragungsmittels (1) mindestens ein auf eindringende Luftfeuchtigkeit ansprechender Sensor (4) vorgesehen ist, der mit einer nachgeschalteten Elektronik (7) derart verbunden ist, daß jede Signal- bzw. Istwertänderung erkannt, ausgewertet und bei Annäherung des Istwertes an einen vorwählbaren Grenzwert der Signalwert oder dessen Differenz zum maximal zuläsigen Grenzwert, in Abhängigkeit eines vorwählbaren Grenzwertbereiches, analog angezeigt und bei Überschreiten des Grenzwertes ein entsprechendes Signal ausgelöst sowie eine mit dem Innenraum des zu überwachenden Aufnahme- oder Übertragungsmittels (1) verbundene Überdruckpumpe (10) zur Konstanthaltung und/oder Reduzierung des Luftfeuchtigkeitgehaltes in Betrieb genommen wird.

2. Bildgebendes Aufnahme- und/oder Übertragungsmittel nach Anspruch 1, dadurch gekennzeichnet, daß der Sensor (4) in unmittelbarer Nähe des Festkörperbildaufnehmers (CCD-Element) angeordnet ist.

3. Bildgebendes Aufnahme- und/oder Übertragungsmittel nach Anspruch 1, dadurch gekennzeichnet, daß mehrere im Abstand zueinder angeordnete, auf Luftfeuchtigkeit ansprechende Sensoren (4) im Innenraum des Aufnahme- und/oder Übertragungsmittels angeordnet sind.

4. Bildgebendes Aufnahme- und/oder Übertragungsmittel nach einem der Ansprüche 1 bis 3, dadurch gekennzeichnet, daß der bzw. die Sensoren (4) einschließlich der nachgeschalteten Auswerteelektronik (7) sowie zur Anzeige von Luftfeuchtigkeitsänderungen und/oder des Überschreitens eines vorwählbaren Luftfeuchtigkeits-Grenzwertes geeignete akustische oder optische Mittel im Innenraum des Aufnahme und/oder Übertragungsmittels angeordnet sind.

## Claims

1. Image-forming recording and/or transmission medium (1), such as an endoscope, a techno-endoscope, a videoscope or an endo-camera, it being possible to connect the sealed cavity thereof to a device for producing excess pressure therein, characterised in that at least one sensor (4) responding to penetrating air moisture is provided in the sealed cavity of the recording and/or transmission medium (1), which sensor (4) is connected to electronics (7) connected downstream such that each signal change or actual value change is recognised, evaluated and when the actual value approaches a pre-selectable limiting value, the signal value or its difference from the maximum permissible limiting value as a function of a pre-selectable limiting value range, is indicated analogously, and when the limiting value is exceeded a corresponding signal is triggered and an excess pressure pump (10) connected to the interior of the recording and/or transmission medium (1) to be monitored is operated to maintain and/or reduce the air moisture content.

2. Image-forming recording and/or transmission medium according to claim 1, characterised in that the sensor (4) is arranged in the immediate vicinity of the solid-state image sensor (CCD element).

3. Image-forming recording and/or transmission medium according to claim 1, characterised in that several sensors (4) responding to air moisture arranged at intervals from one another are arranged in the interior of the recording and/or transmission medium.

4. Image-forming recording and/or transmission medium according to one of claims 1 to 3, characterised in that the sensor or sensors (4) including the downstream evaluation electronics (7), and suitable acoustic or optical means to indicate air moisture changes and/or exceeding a pre-selectable air moisture limiting value, are arranged in the interior of the recording and/or transmission medium.

## Revendications

1. Appareil d'enregistrement et de transmission (1) pourvu d'un dispositif de reproduction d'images, tel qu'un endoscope, un techno-endoscope, viodéoscope ou une endocaméra, dont l'espace creux étanchéifié peut être raccordé à un dispositif de production d'une surpression dans l'espace creux, caractérisé en ce que, dans l'espace creux étanchéifié de l'appareil d'enregistrement et/ou de transmission (1), au moins un capteur (4) sensible à la pénétration de l'humidité de l'air est prévu et relié à une électronique (7) mise en circuit en aval, de manière que chaque variation de signal ou de valeur réelle soit identifié, évalué et que, en cas d'approche de la valeur réelle d'une valeur limite pouvant être préselectionnée, la valeur du signal, ou sa différence par rapport à la valeur limite admissible, est soumise à un affichage analogique, en fonction d'une plage de valeur limite pouvant être préselectionnée, et en cas de dépassement de la valeur limite, un signal correspondant est déclenché ainsi qu'est produit la mise en service d'une pompe de surpression (10), reliée à l'enceinte intérieure de l'appareil d'enregistrement ou de transmission (1) à surveiller, en vue de maintenir la constance de, et/ou de réduire, l'humidité de l'air pendant le fonctionnement.

2. Appareil d'enregistrement et/ou de transmission pourvu d'un dispositif de reproduction d'images selon la revencication 1, caractérisé en ce que le capteur (4) est disposé à proximité immédiate de l'enregistreur d'image à semi-conducteur (élément CCD).

3. Appareil d'enregistrement et/ou de transmission pourvu d'un dispositif de reproduction d'images selon la revendication 1, caractérisé en ce que plusieurs capteurs (4) disposés à distance les uns des autres, sensibles à l'humidité de l'air, sont disposés dans l'enceinte intérieure de l'appareil d'enregistrement et/ou de transmission.

4. Appareil d'enregistrement et de transmission pourvu d'un dispositif de reproduction d'images selon l'une des revendications 1 à 3, caractérisé en ce que le ou les capteurs (4), y compris l'électronique d'évaluation (7) mise en circuit en aval, ainsi que des moyens acoustiques ou optiques, appropriés pour afficher des fluctuations de l'humidité de l'air et/ou le dépassement d'une valeur limite pouvant être présélectionnée de l'humidité de l'air, sont disposés dans l'enceinte intérieure de l'appareil d'enregistrement et/ou de transmission.
